# EUROPEAN PATENT APPLICATION

(11) **EP 4 616 888 A1**
(43) Date of publication of application: **17.09.2025**
(21) Application number: 24205618.2
(22) Date of filing: 09.10.2024
(51) Int. Cl.: A61M 5/32, A61M 5/20

(54) **SAFETY MECHANISM FOR A DRUG DELIVERY DEVICE**

(71) Applicant: Ypsomed AG, 3401 Burgdorf (CH)
(72) Inventor: Loser, Peter, 3037 Herrenschwanden (CH); Bernhard, Mario, 3400 Burgdorf (CH); Wegner, Stephan, 4051 Basel (CH); Shepherd, David, EH12 7HW Edinburgh (GB); Fischbacher, Peter Alastair, EH11 2LX Edinburgh (GB); McLusky, James Donald, EH6 4QN Edinburgh (GB)

(57) **Abstract**

The present invention relates to a safety mechanism for a drug delivery device for dispensing a liquid drug from a reservoir through a needle, the safety mechanism comprising a drive unit and a reservoir unit adapted to hold the reservoir and releasably attachable to the drive unit. The drive unit comprises a drive unit housing defining a longitudinal axis, a plunger movable inside the drive unit housing in a dispensing direction to dispense the liquid drug and a mechanical drive, preferably a compression spring, arranged inside the drive unit housing for moving, when loaded, the plunger in the dispensing direction. The reservoir unit comprises a reservoir unit housing and a cap releasably attachable to a distal end portion of the reservoir unit housing, wherein the cap comprises a deflectable holding member adapted to hold the cap on the distal end portion. The reservoir unit is insertable into the drive unit housing from a distal end of the drive unit housing until it reaches an end position within the drive unit housing. According to the invention the drive unit housing comprises an actuation member adapted to elastically deform the holding member of the cap in a circumferential direction, when the reservoir unit reaches the end position, such that the cap is released from the distal end portion.

## Description

### FIELD OF THE INVENTION

The present invention relates to drug delivery devices or medicament delivery devices for injecting, delivering, administering, infusing or dispensing substances and/or liquids such as insulin, hormone preparations or vaccines. It departs from a drug delivery device comprising a preferably reusable drive unit and a preferably disposable reservoir unit configured for releasable attachment to the drive unit.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a stopper in a container barrel, wherein the drive spring may have to be charged or strained manually prior to injection of a dose. A manually powered delivery drive requires a user to manually provide the energy to move the stopper, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the stopper manually or by pressing a release button of an automatic injection device. Automatic injection devices may comprise an electronic dose dial mechanism to automatically set a dose.

Also known is the use of autoinjectors with prefilled syringes. Autoinjectors usually comprise a body for housing a syringe as well as a drive mechanism in order to move the stopper of the syringe upon actuation of the autoinjector. The drive mechanism typically comprises a source of drive, such as an electric motor or a mechanical spring for moving a transfer element, for example a plunger rod, which acts on the stopper of the syringe. The prefilled syringe has a needle or cannula that is permanently attached to a first end of a syringe barrel or reservoir that is sealed at the opposing end by the stopper.

For safety and hygiene reasons it is desirable that the needle does not protrude from a housing of the autoinjector except for the time when the needle is used for injection of a medicament. Thus, either the autoinjector moves the needle out of the housing for the injection and back into the housing after injection or the autoinjector includes a needle cover which may be moved to unsheathe the needle for injection, and which may be moved back to a needle covering position after the injection.

The majority of autoinjectors are configured as single use devices which incorporate both the syringe and the drive mechanism in the same housing. Such devices are usually disposable for hygiene reasons. Disposable autoinjectors comprising an electric actuator or an electronic control require a source of energy which is usually in the form of a battery. However, in this case, the autoinjectors should not be disposed in the regular waste but have to be subjected to special disposal or to recycling, which is an additional burden to the patient. Further, disposing a battery, motor and/or electronics after a single use is a waste of resources and increases the costs of the therapy. Disposable autoinjectors comprising a mechanical drive, like a compression spring, are easier to dispose than autoinjectors with an electric drive, however it is also a waste of resources to dispose the compression spring after a single use, which also increases the costs of the therapy.

In order to account for a need to handle and dispose the autoinjector parts differently semi-reusable autoinjectors have been developed. Such autoinjectors typically comprise a reusable drive unit and a disposable syringe unit which may be releasably coupled or attached to the drive unit. The drive unit usually includes the drive mechanism and electronics whereas the syringe unit includes the syringe with the needle and a needle cover sleeve. The user can thus discard the syringe unit when it is empty or after use and can load the drive unit with a new syringe unit for a new upcoming injection.

A problem for semi-reusable autoinjectors is that the user has to grab the reservoir unit to attach it to the drive unit before the injection and to separate it from the drive unit after the injection. This leads to an increased risk for the user of accidentally touching the needle and injuring himself. Therefore, it is particularly important that the needle is securely covered before and after the injection.

In many drug delivery devices known from the prior art, for example from CN 217489449 U, EP 2618870 A1, WO 18091916 A1 and US 2016/354556 A1, the syringe is movable relative to the housing of the drug delivery device, so that the needle only protrudes from the housing during the injection and is retracted inside the housing before and after the injection. This prevents needle stick injuries but has the disadvantage that additional components are required in the reservoir unit to enable the syringe movement even though the reservoir unit is disposed after a single use.

WO 14029621 A1 discloses a drug delivery device with a needle shield that covers the needle before the injection. The needle shield can be removed with a specially designed needle shield remover. This reliably prevents the user from accidentally touring the needle but has the disadvantage that the user needs to carry the needle shield remover and cannot use the drug delivery device without the needle shield remover. The drug delivery device also comprises a needle hider body that is movable with respect to the drug delivery housing to cover and uncover the needle. Another disadvantage is that the needle hider front is still movable after the injection, so that the user can accidentally touch the needle when he accidentally pushes the needle hider body which uncovers the needle.

US 2016/175524 Al discloses a drug delivery device in which the needle is covered by a protective cap. The cap can be removed at any time by the user or for example during transport of the drug delivery device, hence also before the reservoir unit is inserted into the drive unit. In this case the needle is uncovered so that the user can accidentally touch the needle.

US 2023/0173186 A1 discloses a drug delivery device with a cap that is locked to the reservoir unit before the reservoir unit is attached to the drive unit. When the reservoir unit is attached to the drive unit, a protrusion on the cap is deflected radially outwards by the drive unit so that the cap can be removed. This exposes a trigger sleeve that still covers the needle but can be moved to uncover the needle by pressing the drug delivery device against the skin. After the injection, the trigger sleeve is still movable, so that the user can accidentally touch the needle after the injection while separating the reservoir unit and the drive unit.

### DESCRIPTION OF THE INVENTION

It is an objective of the present invention to provide a drug delivery device with a mechanical drive in which the needle is securely covered before and after the injection, so that the user cannot accidentally touch the needle.

This objective is achieved by the features of the independent claims. Preferred embodiments are evident from the dependent claims.

In a first aspect, the invention relates to a safety mechanism for a drug delivery device for dispensing a liquid drug from a reservoir through a needle.

The safety mechanism comprises a drive unit and a reservoir unit adapted to hold the reservoir and releasably attachable to the drive unit. With the safety mechanism it is provided that the user cannot accidentally touch the needle, particularly before the injection while attaching the reservoir unit to the drive unit. The drug delivery device is preferably an injection device and more preferably an autoinjector, that is adapted to dispense the whole content of the reservoir in a single injection stroke. Most preferably, the drug delivery device is a semi-disposable autoinjector with a reusable drive unit and a disposable reservoir unit. After each injection the empty reservoir unit is disposed and replaced by a new disposable reservoir unit. The reservoir may be a prefilled syringe having a needle or cannula that is permanently attached to a first end of a syringe barrel that is sealed at the opposing end by a stopper. Alternatively, the reservoir may be a cartridge with a connecting portion at the distal end adapted to be connected to an injection needle before the injection.

The drive unit of the safety mechanism comprises a drive unit housing defining a longitudinal axis, a plunger movable inside the drive unit housing in a dispensing direction to dispense the liquid drug. The plunger can also be referred to as piston or drive element. The longitudinal axis defines a longitudinal direction of the drive unit. The longitudinal axis of the drive unit is also considered as longitudinal axis of the drug delivery device. The dispensing direction can preferably be the longitudinal direction and more preferably a distal direction. The drive unit housing can comprise only one part with a sleeve structure. The drive unit housing can also comprise two or more parts that together form a sleeve structure. In this alternative, the two or more drive unit housing parts can be connected mechanically, for example with screws or latches, and/or they can be bonded to each other, for example by gluing or welding. The drive unit further comprises a mechanical drive arranged inside the drive unit housing for moving, when loaded, the plunger in the dispensing direction. Preferably, the mechanical drive is a compression spring. For loading, the mechanical drive is preferably compressed in a proximal direction, hence a distal end of the mechanical drive is moved in the proximal direction while the proximal end of the mechanical drive is fixed.

The reservoir unit of the safety mechanism comprises a reservoir unit housing. The reservoir unit housing also defines a longitudinal axis and a longitudinal direction of the reservoir unit, that coincides with the longitudinal axis and the longitudinal direction of the drive unit, when the reservoir unit is inserted into the drive unit housing. Preferably, the reservoir unit housing is adapted to hold the reservoir. However, in other alternatives the reservoir unit can further comprise a reservoir holder arranged inside the reservoir unit housing that holds the reservoir. Preferably, after assembly of the reservoir unit, the reservoir is fixed or non-movably arranged inside the reservoir unit housing or the reservoir holder. That means during an injection the reservoir does not move relative to the reservoir unit housing or the reservoir holder and hence also does not move relative to the drive unit housing. This means that the user has to insert or penetrate the injection needle into the injection site by a manual insertion force.

The reservoir unit further comprises a cap releasably attachable to a distal end portion of the reservoir unit housing. The cap comprises an elastically deformable holding member adapted to hold the cap on the distal end portion. Preferably, the holding member is adapted to hold the cap on the distal end portion when the holding member is undeformed and to release the cap from the distal end portion when the holding member is elastically deformed.

The reservoir unit is insertable into the drive unit housing from a distal end of the drive unit housing until it reaches an end position within the drive unit housing. In the sense of the present invention, the end position is a predefined end position, meaning that it is a specific position inside the drive unit housing. For defining the predefined end position, the drive unit housing can comprise a first connection element and the reservoir unit housing can comprise a second connection element, that are adapted to attach or connect the reservoir unit to the drive unit in the end position. The first connection element and the second connection element have to be adapted to be able to provide the connection between the reservoir unit and the drive unit against the force of the loaded mechanical drive. Preferably, the mechanical drive is loaded by inserting the reservoir unit into the drive unit housing. Preferably the drive unit is inserted by a user prior to an injection to assemble the drug delivery device and to prepare the drug delivery device for the injection. This means the drug delivery device is not fully assembled by the manufacturer before the reservoir unit and the drive unit are delivered to the user as individual components.

According to the first aspect of the present invention, the drive unit housing comprises an actuation member adapted to elastically deform the holding member of the cap in a circumferential direction or with other words sideways, when the reservoir unit reaches the end position, such that the cap can be separated from the distal end portion of the reservoir unit housing. The circumferential direction is oriented perpendicular to the axial direction and perpendicular to the radial direction. With other words the circumferential direction can be oriented tangential to the outer contour of the cap respectively the reservoir unit respectively the drug delivery device. The actuation member is adapted to contact the holding member when the reservoir unit reaches the end position or shortly before the reservoir unit reaches the end position to be able to deform the holding member in the end position. When the holding member is deformed, the user can easily separate the cap from the reservoir unit respectively from the drug delivery device by pulling the cap in the distal direction and/or by turning the cap.

An advantage of the safety mechanism according to the invention is that the holding member is automatically deformed so that the cap can be separated from the reservoir unit housing when the reservoir unit reaches its end position. This means that before the reservoir unit reaches the end position, with other words is fully inserted into the drive unit housing, the cap cannot be separated from the reservoir unit housing so that the user cannot accidentally touch the needle, but the user is not required to perform an additional handling step or to carry an additional item to be able to remove the cap. Another advantage is that it is easier for the user to remove the cap, because the user does not have to overcome a holding force between the cap and the reservoir unit once the reservoir unit has reached the end position.

The injection device may be either a manually actuated or an automatic delivery device. Automatic devices or autoinjectors typically include automatic drive means such as an elastic element (for example a pre-tensioned spring) or an electric motor to drive a dispensing member to dispense the liquid drug form the reservoir. Manual delivery devices include a manual drive such as a dispensing button that has to be pressed by the user and by a human force to move the dispensing member in dispensing direction.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The term "distal" is meant to refer to the direction or the end of the drug delivery device carrying an injection needle or an injection cannula, whereas the term "proximal" is meant to refer to the opposite direction or end pointing away from the needle or cannula.

The term "injection device" or "injector" refers to a device that is removed from the injection site after each medication event or drug delivery process, whereas the term "infusion device" refers to a device with a cannula or needle that remains in the skin of the patient for a prolonged period of time, for example, several hours.

In the claims, the word "comprising" or "comprises" does not exclude other elements or steps, and the indefinite article "a" or "an" does not exclude a plurality. For example, "an arm" does not exclude the fact that there may be two or more arms that functionally or structurally fulfill the purpose of "an arm". The mere fact that certain elements or steps are recited in distinct claims shall not preclude the existence of further meaningful combinations of these elements or steps.

In a preferred embodiment, the reservoir unit is insertable into the drive unit housing by a turning movement around the longitudinal axis, preferably by screwing the reservoir unit into the drive unit housing. Preferably, the reservoir unit can also be separated from the drive unit housing by a turning movement around the longitudinal axis, preferably by screwing. The advantage of this preferred embodiment is that the turning movement allows to deform the holding member in the circumferential direction in an easy way. This allows for a simple design of the drug delivery device. Another advantage is that due to the force ratio resulting of the transmission of the rotational movement of the reservoir unit to a longitudinal movement of the plunger to load the mechanical drive, a smaller force exerted by the user is sufficient to load the mechanical drive.

In another preferred embodiment, the holding member extends from a distal end portion of the cap in a proximal direction. Preferably the holding member is formed by two or more holding member arms that are spaced apart from each other in the circumferential direction, preferably equally spaced apart from each other. Preferably, the holding member respectively the two or more holding member arms is/are formed between two slits formed in a circumferential wall of the cap. This allows for an easy implementation of the holding member into the cap without the need of expensive and time-consuming manufacturing steps or complex additional features of the cap.

In another preferred embodiment, the reservoir unit housing comprises a channel through which the holding member extends, when the cap is attached to the reservoir unit housing. The channel can be open towards the outer contour of the reservoir unit housing, hence it can be a recess or groove, or alternatively the channel can be closed in the radial direction. This reduces the dimensions of the cap in the radial direction and hence allows for a compact design of the cap and the drug delivery device. The channel has to be large enough to allow the deformation of the holding member in the circumferential direction.

In another preferred embodiment, the holding member comprises a protrusion on a free proximal end of the holding member and the reservoir unit housing comprises a projection on a distal end portion of the reservoir unit housing, preferably at a proximal end of the channel, adapted to establish a connection between the cap and the reservoir unit until the reservoir unit reaches its end position. The protrusion on the holding member and the protrusion on the reservoir unit housing are in contact with each other when the holding member is in the undeformed state and are separated from each other when the holding member is deformed. This provides an easy way to attach the cap to the distal end portion and to release it from the distal end portion.

In another preferred embodiment, the actuation member is a projection on an inner surface of the drive unit housing at a distal end of the drive unit housing comprising a lateral actuation surface adapted to elastically deform the holding member. When the reservoir unit reaches its end position, the actuation surface pushes the holding member in the circumferential direction. In particular, the projection further comprises a ramped distal guiding surface. The guiding surface can be ramped to be compatible with a thread of the reservoir unit housing.

In another preferred embodiment, the drive unit housing comprises a projection on an inner surface of the drive unit housing and/or the plunger comprises a projection on an outer surface of the plunger, adapted to hold the plunger inside the drive unit housing, before the reservoir unit is inserted. The projection on the plunger and/or the drive unit housing define a most distal position of the plunger. Hence, the plunger cannot move further in the distal direction which would lead to the plunger falling out of the drive unit housing.

In another preferred embodiment, the reservoir unit housing comprises a first thread and the drive unit housing comprises a second thread, wherein the first or second thread comprises a detent and the other of the first or second thread comprises a recess in the thread adapted to receive the detent in the end position of the reservoir unit so that a connection between the reservoir unit and the drive unit is established. This connection holds the reservoir unit inside the reservoir unit against the loaded mechanical drive that exerts a force on the drive unit housing in the longitudinal direction. The connection can additionally be used to provide tactile feedback to the user that the reservoir unit has reached its end position. To separate the reservoir unit from the drive unit, the user has to overcome the holding force by screwing the reservoir unit out of the drive unit housing. However, the holding force is smaller for the turning movement than for the longitudinal movement, so that the user is able to screw the reservoir unit out of the drive unit housing even though the force of the loaded mechanical drive cannot push the reservoir unit out of the drive unit housing.

In another preferred embodiment, the reservoir unit housing and/or the cap comprises a grip surface adapted to be grippable by the user when the user inserts the reservoir unit into the drive unit housing. This makes it easier for the user to screw the reservoir unit in and out of the drive unit housing, because user has a better grip and can therefore better exert a higher force in order to screw the reservoir unit in and out of the drive unit housing. To use the cap as additional grip surface, the cap has to be non-rotatably connected to the reservoir unit housing, for example by the holding member.

In a second aspect, the present invention further relates to a second safety mechanism for a drug delivery device for dispensing a liquid drug from a reservoir through a needle. The second aspect can be combined with the first aspect and the preferred embodiments of the first aspect.

The second safety mechanism also comprises a drive unit and a reservoir unit adapted to hold the reservoir and releasably attachable to the drive unit. With the second safety mechanism it is also provided that the user cannot accidentally touch the needle, particularly after the injection while separating the reservoir unit from the drive unit.

The drive unit of the second safety mechanism comprises a drive unit housing defining a longitudinal axis, a plunger movable inside the drive unit housing in a dispensing direction to dispense the liquid drug and a mechanical drive, preferably a compression spring, arranged inside the drive unit housing for moving, when loaded, the plunger in the dispensing direction. The reservoir unit of the second safety mechanism comprises a reservoir unit housing. For further details regarding the reservoir unit and the drive unit, reference is made to the explanations regarding the first aspect, which apply in the same way for the second aspect.

The reservoir unit of the second safety mechanism further comprises a needle cover or cover member movable inside the reservoir unit housing between an initial position, in which the needle cover covers the needle, and a retracted position, in which the needle protrudes from a distal end of the needle cover. The initial position is distal from the retracted position, meaning that the needle cover has to be moved in the proximal direction to reach the retracted position. Preferably, the needle cover is biased in the distal direction, hence towards the initial position by a needle cover spring. The needle cover is movable along the longitudinal axis and is guided by the reservoir unit housing. Preferably, the needle cover can be movable inside the reservoir unit housing, with other words be enclosed by the reservoir unit housing. The needle cover may be implemented as shield or sleeve or sleeve-shaped element adapted to cover or envelop the needle of the reservoir mounted inside the reservoir unit housing. The needle cover is preferably adapted to release the plunger to move in the dispensing direction, when the needle cover reaches the retracted position or shortly before reaching the retracted position. This means that the injection can be started by pressing the needle cover against the skin at the injection site which pushes the needle cover in the proximal direction relative to the reservoir unit housing to the retracted position.

The reservoir unit is insertable into the drive unit housing from a distal end of the drive unit housing. For further details regarding the insertion of the reservoir unit into the drive unit, reference is made to the explanations regarding the first aspect, which apply in the same way for the second aspect.

According to the second aspect of the present invention, the drive unit of the second safety mechanism comprises a gear element adapted to rotate inside the drive unit housing, when the plunger moves in the dispensing direction. With other words, the gear element is adapted to rotate during the injection. Preferably, the gear element only rotates and does not move in the distal direction or the proximal direction. While rotating, the gear element elastically deforms a needle cover clip of the needle cover in a circumferential direction, or with other words sideways, so that the needle cover can move to a final extended position that is distal from the initial position. The needle cover clip is adapted to prevent a movement of the needle cover in the distal direction past the initial position in an undeformed state of the needle cover clip by abutting a stop surface of the drive unit or the reservoir unit. For example, the stop surface is formed on the reservoir unit housing, preferably at a proximal end portion of the reservoir unit housing. The gear element deforms the needle cover clip so that it no longer abuts the stop surface and is free to move to the final extended position distal from the initial position. Preferably, in the final extended position, the needle cover is locked so that it cannot be pushed back in the proximal direction to reveal the needle. The locking can for example be achieved by an elastically deformable lockout clip of the needle cover that abuts a distal end surface of the reservoir unit housing or a locking protrusion on an inner surface of the reservoir unit housing, when the needle cover is in the final extended position.

An advantage of the second safety mechanism according to the invention is that the needle cover clip is automatically deformed during the injection, so that the needle cover can move to the final extended position after the injection is complete. In the final extended position, the needle cover protrudes further from the distal end of the reservoir unit housing, so that it better covers the needle. Preferably, as described above, the needle cover is additionally locked in the final extended position. Hence, when the user separates the reservoir unit from the drive unit after the injection, the needle is securely covered by the needle cover in the final extended position so that the user cannot accidentally touch the needle. Therein, the user is not required to perform an additional handling step to bring the needle cover in the final extended position, the user simply has to remove the drug delivery device from the skin.

In a preferred embodiment, the gear element comprises a projection on an inner surface of the gear element, preferably formed by a pin connected to the gear element, and the plunger comprises an inner rod with a guiding channel adapted to guide the projection of the gear element so that the gear element rotates. This provides a simple and easy to manufacture way to realize the rotation of the gear element without any additional components or complex manufacturing, assembly and/or handling steps. Preferably, the inner rod section of the plunger is further adapted to contact and move a stopper of the reservoir for dispensing the medicament.

Preferably, the guiding channel on the inner rod section comprises a proximal section and a distal section extending in a longitudinal direction and offset to each other in the circumferential direction. The proximal section and the distal section are connected by a ramped section adapted to rotate the gear element. With other words, the rotation of the gear element is achieved in that the pin of the guiding element is moved from the proximal section to the distal section via the ramped section.

In a preferred embodiment, the gear element comprises a lateral guide surface with a groove for guiding the needle cover clip. The needle cover clip can slide in the groove on the lateral guide surface, so that the needle cover is securely moved to the final extended position without rotation or tilting of the needle cover.

In a preferred embodiment, the needle cover comprises a lockout clip on an outer surface of the needle cover adapted to lock the needle cover in the final extended position by abutting a projection on an inner surface of the reservoir unit housing.

The invention also relates to a drug delivery device comprising a first safety mechanism as described above and/or a second safety mechanism as described above. Preferably, the drive unit housing, the reservoir unit housing, the plunger, the cap, the needle cover and/or the gear element is an injection-molded thermoplastic plastic part. The injection-molded parts allow for a cost-effective production of the reservoir unit parts, in particular in case of a high-volume production.

### BRIEF DESCRIPTION OF THE DRAWINGS

The subject matter of the invention will be explained in more detail in the following text with reference to preferred example embodiments which are illustrated in the attached drawings, in which depict:
- Fig. 1: an example embodiment of an inventive drug delivery device before the reservoir unit is inserted into the drive unit;
- Fig. 2: the drug delivery device of fig. 1 after the reservoir unit is inserted into the drive unit;
- Fig. 3: an exploded view of the drug delivery device of fig. 1 and 2;
- Fig. 4: an exploded view of the reservoir unit of the drug delivery device of fig. 1 to 3;
- Fig. 5: a sectional view of the drive unit of the drug delivery device of fig. 1 to 4 before the reservoir unit is inserted into the drive unit;
- Fig. 6: a sectional view of the reservoir unit of the drug delivery device of fig. 1 to 4 before the reservoir unit is inserted into the drive unit;
- Fig. 7: a side view of the reservoir unit of fig. 6 before the reservoir unit is inserted into the drive unit;
- Fig. 8: the reservoir unit of fig. 6 and 7 in a perspective view;
- Fig. 9: the cap of the reservoir unit of fig. 6 to 8 in a perspective view;
- Fig. 10: a sectional view of the drug delivery device of fig. 1 to 4 while the reservoir unit is inserted into the drive unit;
- Fig. 11: a perspective view of the plunger of the drug delivery device of fig. 1 to 4;
- Fig. 12: a perspective view of the drive unit housing of the drug delivery device of fig. 1 to 4;
- Fig. 13: the distal end of the drug delivery device of fig. 1 to 4 after the reservoir unit is inserted into the drive unit;
- Fig. 14: a sectional view of the drug delivery device of fig. 1 to 4 with the needle cover in the initial position;
- Fig. 15: the drug delivery device of fig. 14 with the needle cover in the retracted position;
- Fig. 16: the drug delivery device of fig. 14 and 15 before injection;
- Fig. 17: the drug delivery device of fig. 14 to 16 during injection;
- Fig. 18: the drug delivery device of fig. 14 to 17 during injection;
- Fig. 19: the plunger and the gear unit of the drug delivery device of fig. 14 to 18 before, during and after injection;
- Fig. 20: two sectional views of the drug delivery device of fig. 14 to 19 with the cover sleeve in the initial position;
- Fig. 21: two sectional views of the drug delivery device of fig. 14 to 20 with the cover sleeve in the retracted position before injection;
- Fig. 22: two sectional views of the drug delivery device of fig. 14 to 21 with the cover sleeve in the retracted position after injection; and
- Fig. 23: two sectional views of the drug delivery device of fig. 14 to 22 with the cover sleeve in the distal position.

The reference symbols used in the drawings, and their primary meanings, are listed in summary form in the list of designations. In principle, identical parts are provided with the same reference symbols in the figures.

### DETAILED DESCRIPTION OF THE PREFFERRED EMBODIMENTS

In the present description the term "distal" refers to the side where the injection needle is located. This is on the right side or the lower part of the figures. The term "proximal" refers to the opposite side or rear end of the device. This is on the left side or the upper part of the figures.

The figures show an example embodiment of a drug delivery device 1 according to the present invention in form of a semi-reusable autoinjector with a first safety mechanism and a second safety mechanism according to the present invention.

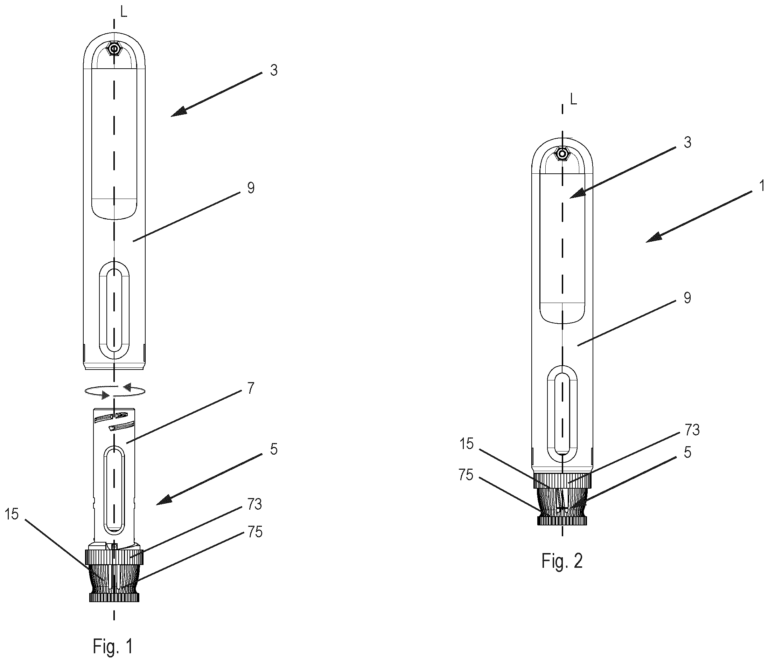

The autoinjector 1 comprises a reusable drive unit 3 and a disposable reservoir unit 5 releasably attachable or connectable to the drive unit 3. The reservoir unit 3 can be inserted into the drive unit 5 from a distal end of the drive unit 3 by screwing the reservoir unit 5 into the drive unit 3 until it reaches an end position within the drive unit 3. This means the reservoir unit 5 is insertable by a turning movement around a longitudinal axis L defined by the drive unit 3, as indicated by the arrow in fig. 1.

Fig. 1 shows the autoinjector before the reservoir unit 5 is inserted in the drive unit 3 and fig. 2 shows the fully assembled autoinjector 1 after the reservoir unit 5 is inserted into the drive unit 3. The drive unit 3 of the second example embodiment has length of 159 mm in the longitudinal direction and an elliptical cross section with outer dimensions of 28 mm and 29 mm, hence and almost circular cross section. The reservoir unit 5 of the second example has a length of 90.3 mm and a circular cross section with a diameter of 27.2 mm. The assembled autoinjector 1 as shown in fig. 2 has a total length of 182.5 mm, hence a major part of the reservoir unit 5 is inserted into the drive unit 3. This allows for a compact design of the autoinjector 1.

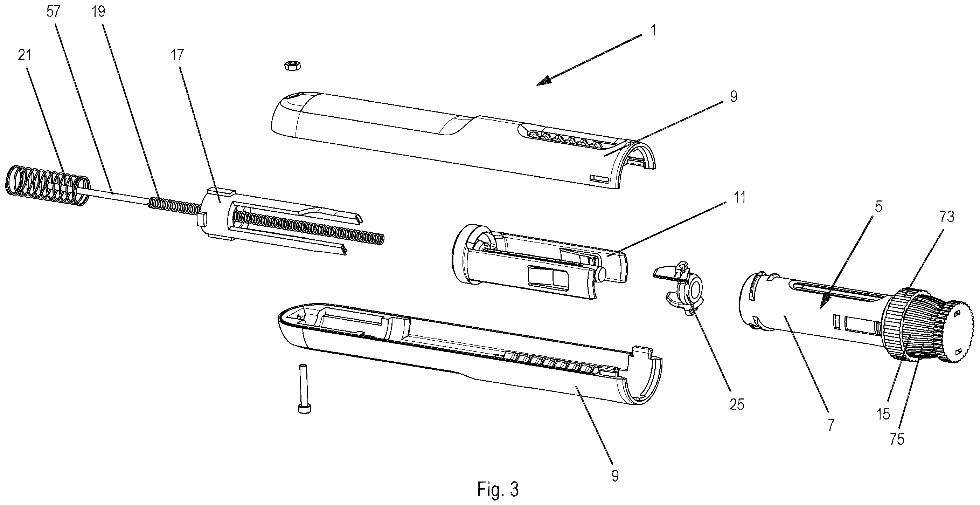

Fig. 3 shows the autoinjector 1 in an exploded view that gives an overview of the separate components of the drive unit 3 of the autoinjector 1. The main components of the drive unit 3 are a drive unit housing 9 defining the longitudinal axis L, a plunger 11 movable inside the drive unit housing 9 in a dispensing direction to dispense the liquid drug, a pusher sleeve 17, a mechanical drive 19 in form of a compression spring, a pusher sleeve spring 21 also in form of a compression spring and a gear unit 25. The gear unit 25 rotates during the injection and provides a lockout of the needle cover 13 in a final extended position of the needle cover 13 in which the needle is fully covered by the needle cover 13. The drive unit housing 9 comprises two housing parts that are connectable to each other by latches on the distal end of the drive unit housing 9 and a screw and nut connection on a proximal end of the drive unit housing 9. The drive unit 3 also comprises a mechanical drive rod 57 that is arranged inside the mechanical drive 19. The mechanical drive rod 57 prevents the mechanical drive 19 from bending during compression and relaxation of the mechanical drive 19.

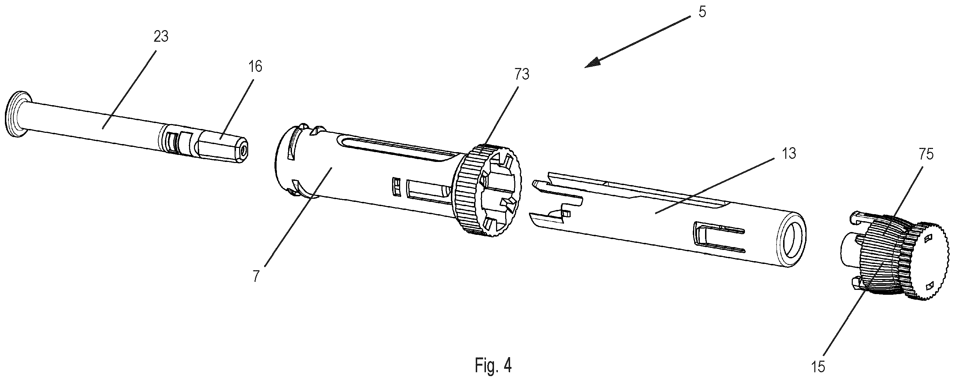

Fig. 4 shows the reservoir unit 5 of the autoinjector 1 in an exploded view that gives an overview of the separate components of the reservoir unit 5. The reservoir unit 5 comprises a reservoir unit housing 7, a needle cover 13, a cap 15 releasably attachable to a distal end portion of the reservoir unit housing 7 and a reservoir 23 with an injection needle. The reservoir 23 is a prefilled syringe or cartridge with a stopper at a proximal end and the needle permanently attached to a distal end. The reservoir 23 further comprises a needle shield 16 that covers and protects the needle and that has to be removed before the injection.

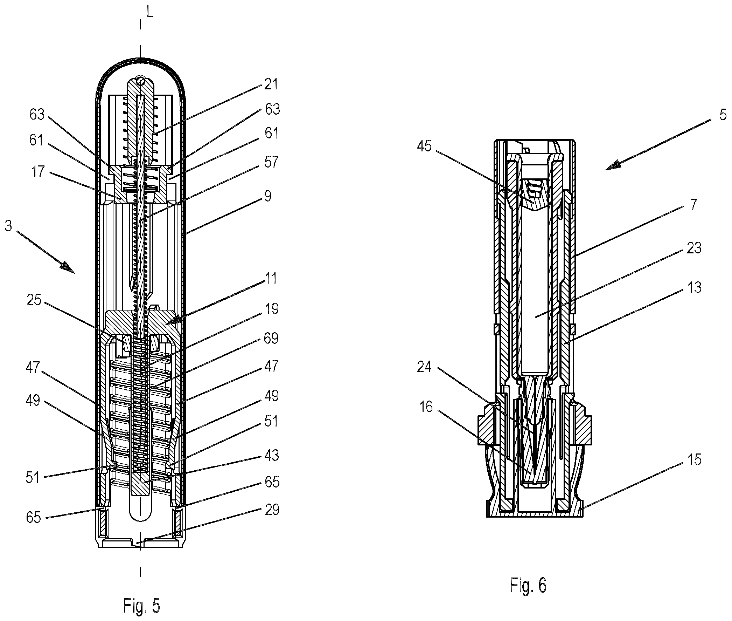

Fig. 5 shows the drive unit 3 in its initial state before the reservoir unit 5 is inserted. The pusher sleeve 17 is prevented from moving in the distal direction by circumferential proximal projections 61 on an inner surface of the drive unit housing 9 that abut a circumferential shoulder 63 of the pusher sleeve 17 and thereby ensure that the pusher sleeve 17 stays inside the drive unit housing 9. The plunger 11 is held inside the drive unit housing 9 and prevented from moving in the distal direction by the plunger arms 47 contacting a circumferential distal projection 65 of the drive unit housing 9. The drive unit 3 can be assembled in that the plunger 11 and the pusher sleeve 17 are placed inside the drive unit housing 9 and after that the two housing halves of the drive unit housing 9 are connected to each other.

Fig. 6 shows the reservoir unit housing 5 in its initial state before it is inserted into the drive unit 3. The cap 15 is locked to the reservoir unit housing 7 until the reservoir unit 5 has reached the end position within the drive unit housing 9, which will be explained in detail below. The needle cover 13 is held in place inside the reservoir unit housing 7 by friction between the needle cover 13 and the reservoir unit housing 7. In other embodiments the reservoir unit housing and/or the needle cover could comprise an additional detent to keep the needle cover in its position.

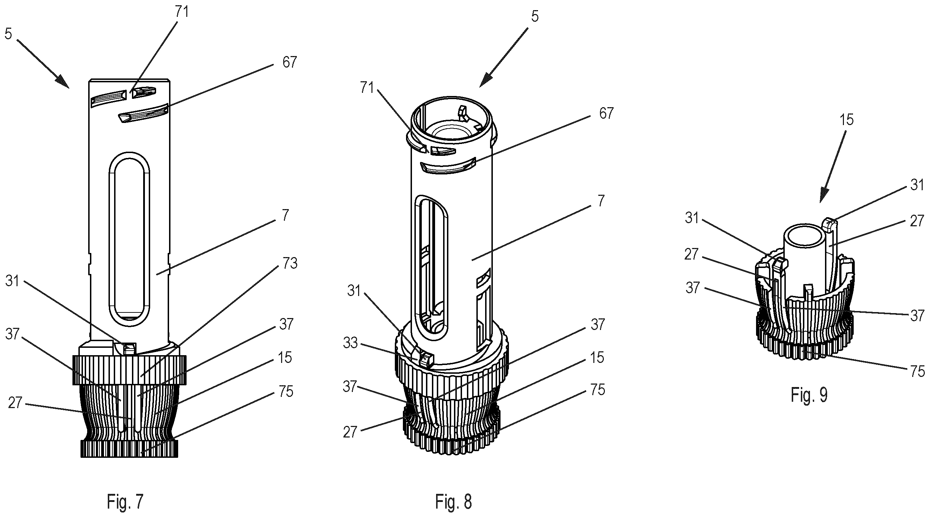

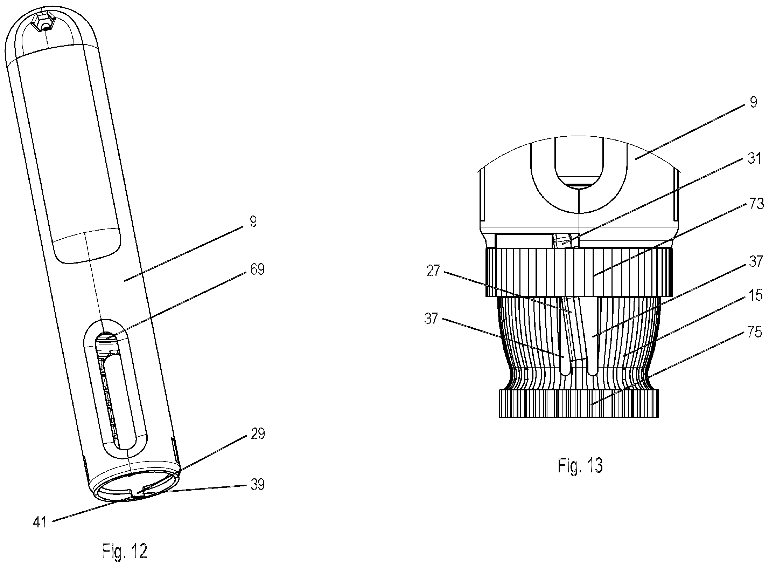

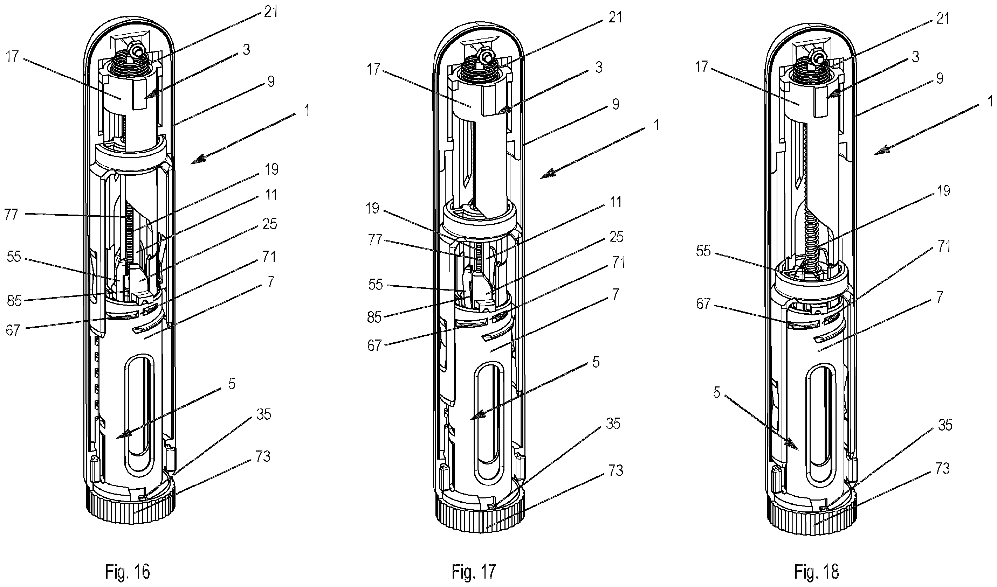

To attach the cap 15 to the reservoir unit housing 7, the cap 15 comprises two elastically deformable holding members 27 arranged opposite to each other, as shown best in fig. 9. The holding members 27 extend from a distal end portion of the cap 15 in a proximal direction. The holding members 27 are each formed by two slits 37 in the circumferential wall of the cap 15, that separate the holding members 27 from the remaining portions of the wall of the cap 15. Due to the small dimension of the holding members 27 in the circumferential direction, the holding members 27 are elastically deformable. The slits 37 also give the holding members 27 space to be deformed in the circumferential direction, as shown in fig. 13. The holding members 27 each comprise a protrusion 31 on a free proximal end of the holding member 27. The reservoir unit housing 7 comprises two channels 33 through which the holding members 27 extend, when the cap 15 is attached to the reservoir unit housing 7. At a proximal end of the channels 33, the reservoir unit housing 7 comprises two projections 35, visible in fig. 16 to 18 in which the cap 15 is removed. The protrusions 31 of the holding members 27 and the projections 35 of the reservoir unit housing 7 establish a connection between the cap 15 and the reservoir unit housing 7 as long as the holding members 27 are in an undeformed state. The protrusions 31 and the projections 35 abut each other, so that the cap 15 cannot be moved in the distal direction and is thereby locked to the reservoir unit housing 7, as shown in fig. 8. To unlock the cap, the reservoir unit 5 has to be screwed into the drive unit housing 9 until it reaches its end position within the drive unit housing 9, which will be explained in detail below.

To be able to screw the reservoir unit 5 into the drive unit 3, the reservoir unit housing 7 comprises a first thread 67, as shown in fig. 7, and the drive unit housing 9 comprises a second thread 69, as shown in fig. 5. The first thread 67 and the second thread 69 are compatible to each other. The second thread 69 of the drive unit housing 9 comprises two thread starts to make it easier to insert the reservoir unit 5 into the drive unit housing 9, because the reservoir unit 5 can be inserted in two different orientations instead of just one orientation. Insertion of the reservoir unit 5 into the distal end of the drive unit housing 9 followed by screwing the reservoir unit 5 into the drive unit housing 9 engages the thread 67 of the reservoir unit housing 7 with the complementary thread 69 of the drive unit housing 9. To make the screwing of the reservoir unit 5 in and out of the drive unit housing 9 easier, the reservoir unit housing 7 comprises a grip area 73 at a distal end of the reservoir unit housing 7 that the user can grip and hold easily and securely. To further facilitate the screwing of the reservoir unit 5, the cap 15 also comprises a grip area 75, so that the grip area is bigger, and it is easier and more comfortable for the user to grip the reservoir unit 5.

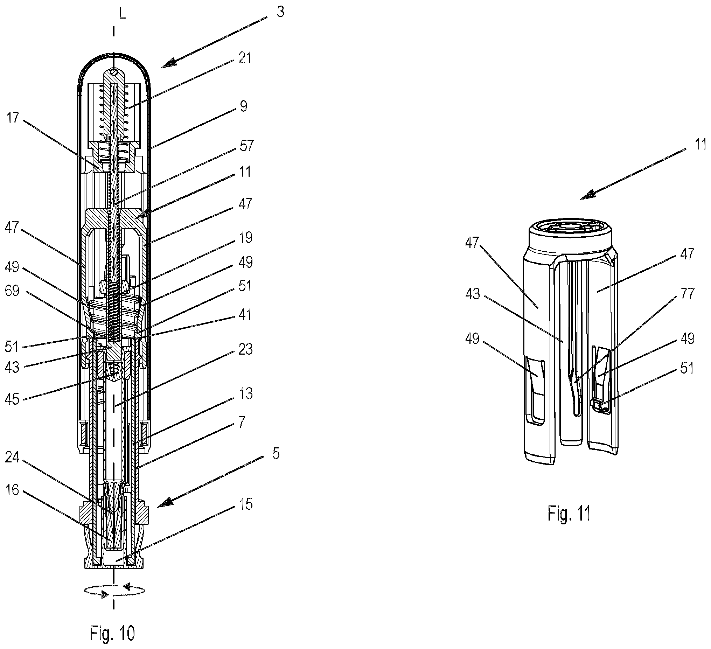

Fig. 10 shows the drive unit 3 and the reservoir unit 5 in a cross-sectional view while the reservoir unit 5 is inserted into the drive unit housing 9 by screwing the reservoir unit 5 into the drive unit housing 9, as indicated by the arrow in fig. 10. While the reservoir unit 5 is inserted into the drive unit housing 9, a proximal end surface of the reservoir unit housing 7 contacts the plunger 11 and pushes the plunger 11 in the proximal direction to load the mechanical drive 19 by compressing the compression spring.

Fig. 11 shows the plunger 11 in detail. The plunger 11 comprises an inner rod section 43 with a distal end surface adapted to contact and move the stopper 45 of the reservoir 23 for dispensing the liquid drug and two plunger arms 47 arranged radially offset from the inner rod section 43 adapted to contact the proximal end surface of the reservoir unit housing 7. The inner rod section 43 has a sleeve-shaped proximal section that defines an interior adapted to receive the mechanical drive 19 therein and forms a proximal end surface adapted to serve as contact surface for the distal end of the mechanical drive 19. Each plunger arm 47 comprises a plunger clip 49 with a projection 51 on its free end. When the reservoir unit 5 is inserted into the drive unit housing 9, the proximal end surface of the reservoir unit housing 7 contacts the projections 51 on the plunger clips 49 to move the plunger 11 in the proximal direction. The plunger clips 49 further serve to lock the plunger 11 before the injection is started and to release the plunger 11 in order to start the injection, which will be explained in detail further below.

Fig. 12 shows the drive unit housing 9 in detail. The drive unit housing 9 comprises two actuation members 29 in form of two protrusions on the inner surface of the drive unit housing at a distal end of the drive unit housing 9. The two actuation members 29 are arranged opposite to each other. The actuation member 29 comes in contact with the holding member 27 and elastically deforms the holding member 27 in a circumferential direction, when the reservoir unit 5 reaches its end position within the drive unit housing 9. Due to the deformation of the holding member 27, that is shown in fig. 13, the cap 15 can be separated or removed from the reservoir unit housing 7, as the protrusion 35 on the reservoir unit housing 7 and the protrusion 31 of the holding member 27 no longer contact each other. Therefore, the cap 15 is free to move in the distal direction. The cap 15 is engaged with the needle shield 16 of the reservoir 23, so that when the cap 15 is removed, the needle shield 16 is removed together with the cap 15. Each actuation member 29 comprises a lateral actuation surface 39 that pushes one of the holding members 27 in the circumferential direction. Each actuation member further comprises a ramped distal guiding surface 41 that is guided by the last thread turn of the thread 67 of the reservoir unit housing 7, shortly before the reservoir unit 5 reaches its end position. The ramped guiding surface 41 ensures that the actuation surfaces 39 are positioned correctly with regard to the holding members 27 to be able to reliably deform the holding members 27.

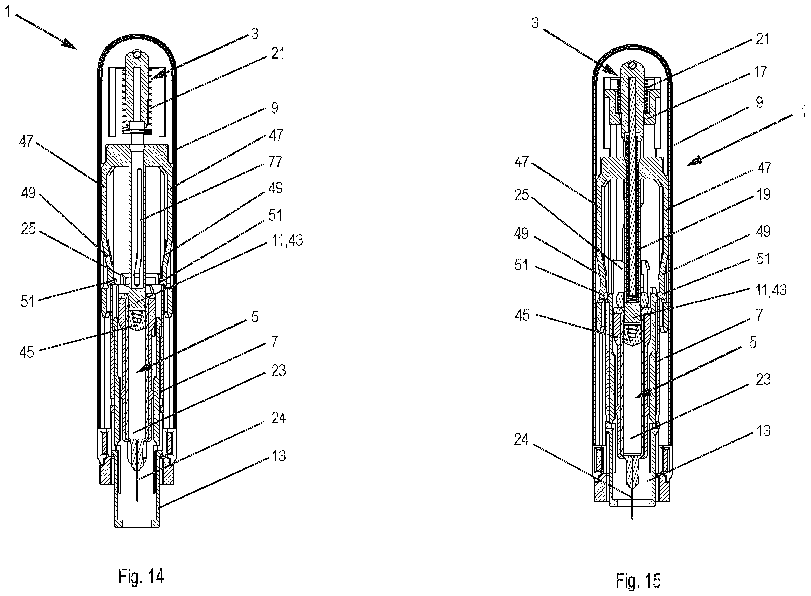

Fig. 14 to 23 show the fully assembled autoinjector 1 when the reservoir unit 5 has reached the predefined end position within the drive unit housing 9. The end position corresponds to a loaded state of the mechanical drive 19 due to a compression of the mechanical drive 19 between a proximal inner end of the drive unit housing 9 and the proximal end surface of the inner rod section 43 of the plunger 11. In the predefined end position, the reservoir unit 5 and the drive unit 3 are connected by a detent on an inner surface of the drive unit housing 9 and a corresponding recess 71 in the thread 67 of the reservoir unit housing 7, as shown for example in fig. 7. In the end position, the detent is received and fixed inside the recess 71, so that the reservoir unit 5 and the drive unit 3 are securely fixed to each other. To release the reservoir unit 5 from the drive unit 3, the user has to manually screw the reservoir unit 5 out of the drive unit housing 9 after the injection is complete.

Fig. 14 shows the autoinjector 1 before the injection with the needle cover 13 in an initial position, in which the needle cover 13 covers the needle 24. In use, the autoinjector 1 is ready to perform an injection after the user has removed the cap 15 from the autoinjector 1. To start the injection, the user presses the autoinjector 1 against the skin at a desired injection site. This causes the needle cover 13 to move from the initial position to a retracted position, in which the needle 24 protrudes form a distal end of the needle cover 13, by moving in the proximal direction with respect to the reservoir unit housing 7 and the reservoir 23. Fig. 15 shows the autoinjector 1 with the needle cover 13 in the retracted position. While the needle cover 13 moves in the proximal direction, the needle cover 13 elastically deforms the plunger clips 49 in the radial direction from the initial position in which the plunger clips 49 hold the plunger 11 against the bias of the loaded mechanical drive 19 to a deformed position. In the deformed position, the plunger 11 is released so that the plunger 11 is free to move in the distal direction under the bias of the loaded mechanical drive 19 to start the injection and to expel the medicament from the reservoir 23. The trigger point for the release of the plunger 11 is shortly before the needle cover 13 reaches its retracted position or proximal position.

After the injection, the needle cover 13 is automatically moved to a final extended position that is distal from the initial position of the needle cover 13, hence the needle cover 13 protrudes further from the distal end of the reservoir unit housing 7 than in the initial position. In the final extended position, the needle cover 13 is locked out, hence it cannot move back in the proximal direction after the needle cover 13 has reached the final extended position. The lockout of the needle cover 13 is provided by the second safety mechanism, which will be explained with reference to fig. 16 to 23.

Fig. 16 shows the autoinjector 1 at the beginning of the injection process, with the plunger 11 in its most proximal position. From this position, the plunger moves in the dispensing direction during the injection to dispense the liquid drug from the reservoir 23, as shown in fig. 17 that shows the plunger 11 in an intermediate position and fig. 18 that the shows the plunger 11 in its most distal position at the end of the injection. In all of the fig. 16 to 18, the needle cover 13 is in the retracted position. By a comparison of the fig. 16 to 18, it is shown that the gear element 25 rotates during the injection process, from a position slightly right of the recess 71 in the thread 67in fig. 16 to a position slightly left of the recess 71 in the thread 67 in fig. 18. Thereby, the gear element 25 elastically deforms needle cover clips 55 in the circumferential direction.

Due to the deformation of the needle cover clips 55, the needle cover 13 is free to move in the distal direction to the final extended position. To guide the needle cover 13, the gear element 25 comprises a lateral guide surface 85 with a groove for guiding the needle cover clip 55.

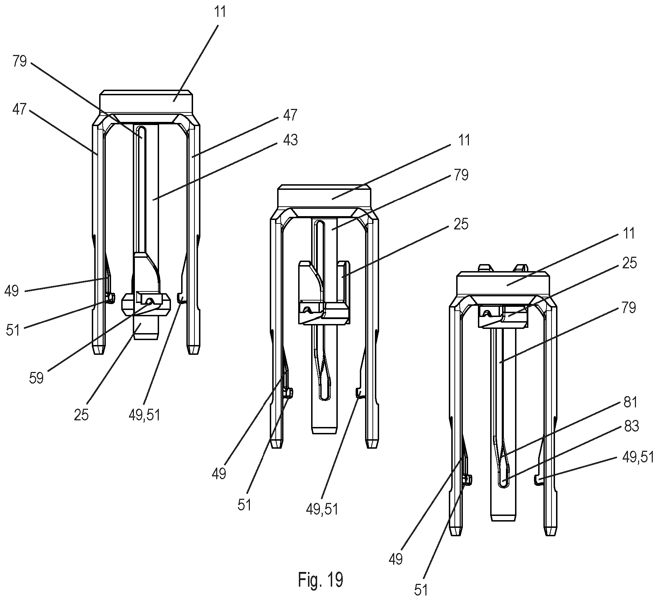

Fig. 19 shows the interaction between the plunger 11 and the gear element 25 and more clearly shows the rotation of the gear element 25 during the movement of the plunger 11. Fig. 19 also shows that the gear element 25 comprises a pin 59 that protrudes from an inner surface of the gear element (not visible in fig. 19). The plunger 11 comprises a corresponding guiding channel 77 on the inner rod section 43 of the plunger that guides the pin 59 and thereby causes the rotation of the gear element 25. To achieve the rotation, the guiding channel 77 comprises a proximal longitudinal section 79 and a distal longitudinal section 81 extending in the longitudinal direction and offset to each other in the circumferential direction. Between the proximal longitudinal section 79 and the distal longitudinal section 81, the guiding channel 77 comprises a ramped section 83, that guides the pin 59 from the proximal longitudinal section 79 to the distal longitudinal section 81, and hence rotates the gear element 25.

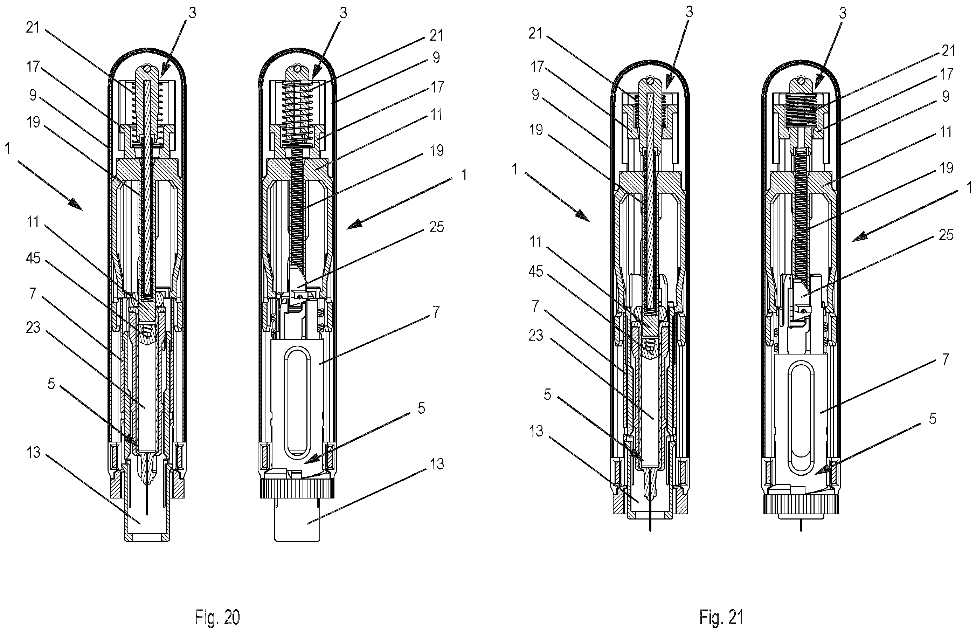

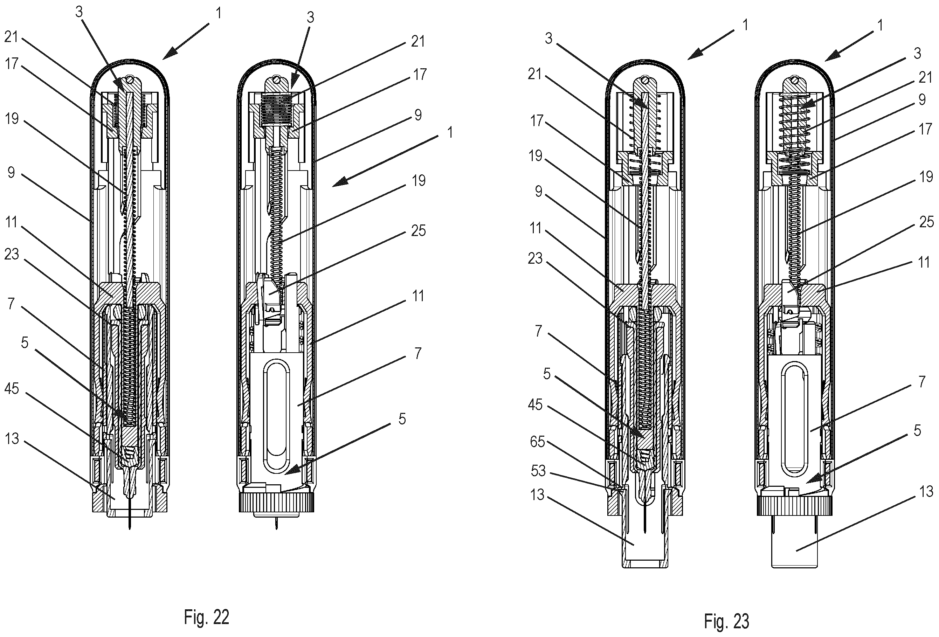

Fig. 20 to 23 again give an overview over the injection process, wherein fig. 20 shows the autoinjector 1 before the injection after the cap 15 was removed, fig. 21 shows the autoinjector 1 with the needle cover 13 in the retracted position, fig. 22 shows the autoinjector1 after the injection is completed with the needle cover 13 still in the retracted position and fig. 23 shows the autoinjector 1 with the needle cover 13 locked in the final extended position. To lock the needle cover 13, the needle cover 13 comprises lockout clips 53 on the outer surface of the needle cover 13, that are in an elastically deformed state until the needle cover 13 reaches the final extended position. In the final extended position, the lockout clips 53 return to an undeformed state and abut the projections 65 on the inner surface of the reservoir unit housing 7, as shown in fig. 23 on the left side.

### LIST OF DESIGNATIONS

- 1: drug delivery device
- 3: drive unit
- 5: reservoir unit
- 7: reservoir unit housing
- 9: drive unit housing
- 11: plunger
- 13: needle cover
- 15: cap
- 16: needle shield
- 17: pusher sleeve
- 19: mechanical drive
- 21: pusher sleeve spring
- 23: reservoir
- 24: needle
- 25: gear element
- 27: holding member
- 29: actuation member
- 31: protrusion
- 33: channel
- 35: projection
- 37: slit
- 39: lateral actuation surface
- 41: distal guiding surface
- 43: inner rod section plunger
- 45: stopper
- 47: plunger arm
- 49: plunger clip
- 51: projection
- 53: lockout clip
- 55: needle cover clip
- 57: mechanical drive rod
- 59: pin
- 61: proximal projection
- 63: shoulder
- 65: distal projection
- 67: first thread
- 69: second thread
- 71: recess
- 73: grip area
- 75: grip area
- 77: guiding channel
- 79: proximal longitudinal section
- 81: distal longitudinal section
- 83: ramped section
- 85: guide surface

- L: longitudinal axis

## Claims

1. A safety mechanism for a drug delivery device (1) for dispensing a liquid drug from a reservoir (23) through a needle (24), the safety mechanism comprising a drive unit (3) and a reservoir unit (5) adapted to hold the reservoir (23) and releasably attachable to the drive unit (3),
the drive unit (3) comprising a drive unit housing (9) defining a longitudinal axis (L), a plunger (11) movable inside the drive unit housing (9) in a dispensing direction to dispense the liquid drug and a mechanical drive (19), preferably a compression spring, arranged inside the drive unit housing (9) for moving, when loaded, the plunger (11) in the dispensing direction,
the reservoir unit (5) comprising a reservoir unit housing (7) and a cap (15) releasably attachable to a distal end portion of the reservoir unit housing (7), wherein the cap (15) comprises an elastically deformable holding member (27) adapted to hold the cap (15) on the distal end portion,
wherein the reservoir unit (5) is insertable into the drive unit housing (9) from a distal end of the drive unit housing (9) until it reaches an end position within the drive unit housing (9),
**characterized in that** the drive unit housing (9) comprises an actuation member (29) adapted to elastically deform the holding member (27) of the cap (15) in a circumferential direction, when the reservoir unit (5) reaches the end position, such that the cap (15) can be separated from the distal end portion.

2. Safety mechanism according to claim 1, wherein the reservoir unit (5) is insertable into the drive unit housing (9) by a turning movement around the longitudinal axis (L), preferably by screwing the reservoir unit (5) into the drive unit housing (9).

3. Safety mechanism according to one of the preceding claims, wherein the holding member (27) extends from a distal end portion of the cap (15) in a proximal direction, wherein in particular the holding member (27) is formed between two slits (37) formed in a circumferential wall of the cap (15).

4. Safety mechanism according to one of the preceding claims, wherein the reservoir unit housing (7) comprises a channel (33) through which the holding member (27) extends, when the cap (15) is attached to the reservoir unit housing (7).

5. Safety mechanism according to one of the preceding claims, wherein the holding member (27) comprises a protrusion (31) on a free proximal end of the holding member (27) and the reservoir unit housing (7) comprises a projection (35) on a distal end portion of the reservoir unit housing (7), preferably at a proximal end of the channel (33), adapted to establish a connection between the cap (15) and the reservoir unit (5) until the reservoir unit (5) reaches its end position.

6. Safety mechanism according to one of the preceding claims, wherein the actuation member (29) is a projection on an inner surface of the drive unit housing (9) at a distal end of the drive unit housing (9) comprising a lateral actuation surface (39) adapted to elastically deform the holding member (27), wherein in particular the projection further comprises a ramped distal guiding surface (41).

7. Safety mechanism according to one of the preceding claims, wherein the drive unit housing (9) comprises a projection (65) on an inner surface of the drive unit housing (9) and/or the plunger (11) comprises a projection on an outer surface of the plunger (11), adapted to hold the plunger (11) inside the drive unit housing (9), before the reservoir unit (5) is inserted.

8. Safety mechanisms according to one of the preceding claims, wherein the reservoir unit housing (7) comprises a first thread (67) and the drive unit housing (9) comprises a second thread (69), wherein the first or second thread (67, 69) comprises a detent and the other of the first or second thread (67, 69) comprises a recess (71) in the thread (67, 69) adapted to receive the detent in the end position of the reservoir unit (5).

9. Safety mechanism according to one of the preceding claims, wherein the reservoir unit housing (7) and/or the cap (15) comprises a grip surface (73, 75) adapted to be grippable by the user when the user inserts the reservoir unit (5) into the drive unit housing (9).

10. A safety mechanism for a drug delivery device (1) for dispensing a liquid drug from a reservoir (23) through a needle (24), the safety mechanism comprising a drive unit (3) and a reservoir unit (5) adapted to hold the reservoir (23) and releasably attachable to the drive unit (3),
the drive unit (3) comprising a drive unit housing (9) defining a longitudinal axis (L), a plunger (11) movable inside the drive unit housing (9) in a dispensing direction to dispense the liquid drug and a mechanical drive (19), preferably a compression spring, arranged inside the drive unit housing (9) for moving, when loaded, the plunger (11) in the dispensing direction
the reservoir unit (5) comprising a reservoir unit housing (7) and a needle cover (13) movable inside the reservoir unit housing (7) between an initial position, in which the needle cover (13) covers the needle (24), and a retracted position, in which the needle (24) protrudes from a distal end of the needle cover (13),
wherein the reservoir unit (5) is insertable into the drive unit housing (9) from a distal end of the drive unit housing (9),
**characterized in that** the drive unit (3) of the safety mechanism comprises a gear element (25) adapted to rotate inside the drive unit housing (9), when the plunger (11) moves in the dispensing direction, wherein while rotating, the gear element (25) elastically deforms a needle cover clip (55) of the needle cover (13) in a circumferential direction, so that the needle cover (13) can move to a final extended position that is distal from the initial position.

11. Safety mechanism according to claim 10, wherein the gear element (25) comprises a projection on an inner surface of the gear element (25), preferably formed by a pin (59) connected to the gear element (25), and the plunger (11) comprises an inner rod section (43) with a guiding channel (77) adapted to guide the projection of the gear element (25) so that the gear element (25) rotates.

12. Safety mechanism according to claim 11, wherein the guiding channel (77) on the inner rod section (43) comprises a proximal section (79) and a distal section (81) extending in a longitudinal direction and offset to each other in the circumferential direction, wherein the proximal section (79) and the distal section (81) are connected by a ramped section (83).

13. Safety mechanism according to one of claims 10 to 12, wherein the gear element (25) comprises a lateral guide surface (85) with a groove for guiding the needle cover clip (55).

14. Safety mechanism according to one of claims 10 to 13, wherein the needle cover (13) comprises a lockout clip (53) on an outer surface of the needle cover (13) adapted to lock the needle cover (13) in the final extended position by abutting a projection (65) on an inner surface of the reservoir unit housing (7).

15. A drug delivery device (1) comprising a safety mechanism according to one of the preceding claims.
